# EUROPEAN PATENT SPECIFICATION

(11) **EP 2 044 209 B1**
(45) Date of publication and mention of the grant of the patent: **08.11.2017**
(21) Application number: 07812331.2
(22) Date of filing: 26.06.2007
(51) Int. Cl.: C12Q 1/00, G01N 27/30, G01N 33/487

(54) **DIAGNOSTIC STRIP WITH CONDUCTIVE LAYERS**
DIAGNOSESTREIFEN MIT LEITENDEN SCHICHTEN
BANDE DIAGNOSTIQUE AVEC COUCHES CONDUCTRICES

(30) Priority: 18.07.2006 US 458298
(43) Date of publication of application: 08.04.2009
(73) Proprietor: NIPRO DIAGNOSTICS, INC., Fort Lauderdale, FL 33309 (US)
(72) Inventor: POPOVICH, Natasha, Pompano Beach, FL 33060 (US); NEEL, Gary, Weston, FL 33327 (US); SLOMSKI, Dennis, Tualatin, Oregon 97062-8386 (US); WEGNER, Greta, Saint Anthony, MN 55418 (US)
(74) Representative: Molnia, David
(86) International application number: PCT/US2007/072123
(87) International publication number: WO 2008/011247

(56) References cited:
- EP-A- 1 288 653
- WO-A-2005/088319
- WO-A-2007/121121
- DE-A1- 10 222 271
- JP-A- 2000 019 147
- JP-A- 2001 311 711
- US-A1- 2005 019 953

## Description

### DESCRIPTION OF THE INVENTION

### Field of the Invention

The present invention relates to electrochemical sensors and, more particularly, to systems and methods for electrochemically sensing a particular constituent within a fluid through the use of diagnostic test strips.

### Background of the Invention

Many industries have a commercial need to monitor the concentration of particular constituents in a fluid. The oil refining industry, wineries, and the dairy industry are examples of industries where fluid testing is routine. In the health care field, people such as diabetics, for example, have a need to monitor a particular constituent within their bodily fluids. A number of systems are available that allow people to test a body fluid, such as blood, urine, or saliva, to conveniently monitor the level of a particular fluid constituent, such as, for example, cholesterol, proteins, or glucose. Patients suffering from diabetes, a disorder in which insufficient insulin production and/or insulin resistance prevents the proper use of glucose by the body, have a need to carefully monitor their blood glucose levels regularly. A number of systems that allow people to conveniently monitor their blood glucose levels are available. Such systems typically include a test strip where the user applies a blood sample and a meter that "reads" the test strip to determine the glucose level in the blood sample.

Among the various technologies available for measuring blood glucose levels, electrochemical technologies are desirable because only a very small blood sample may be needed to perform the measurement. In amperometric electrochemical-based systems, the test strip typically includes a sample chamber that contains electrodes and reagents, such as an enzyme and a mediator. When the user applies a blood sample to the sample chamber, the reagents react with the glucose, and the meter applies a voltage to the electrodes to cause a redox reaction. The meter measures the resulting current and calculates the glucose level based on the current. Other systems based on coulometry or voltametry are also known.

Because the test strip includes a biological reagent, every strip manufactured is not produced with exactly the same sensitivity. Therefore, test strips are manufactured in distinct lots, and data particular to each lot is often used by the meter's microprocessor to assist in meter calibration. The data is used to help accurately correlate the measured current with the actual glucose concentration.

In past systems, the code particular to a specific lot of strips was input into the meter by the user or connected through some type of memory device, such as a ROM chip, packaged along with test strips from a single manufacturing lot. This step of manual input or connection by the user increases the risk of erroneous meter calibration due to human error. Such errors can lead to inaccurate measurements and an improper recording of the patient's glucose level.

Past systems have also included machine-readable information (e.g. bar codes) incorporated onto individual strips. The machine-readable information is interpreted by the meter to provide information related to calibration for an individual test strip. However, individually imprinting a particular bar-code on each strip can add significant cost to strip manufacturing and requires the additional expense and complexity of a bar-code reader incorporated within the meter.

It should be emphasized that accurate measurements of analyte concentrations in a body fluid, such as blood, may be critical to the long-term health of many users. As a result, there is a need for a high level of reliability in the meters and test strips used to measure analyte concentrations in fluids. Thus, it is desirable to have a cost effective auto-calibration system for diagnostic test strips that more reliably and more accurately provides a signaling code for individual test strips.

In addition, in many blood analyte measurement systems, a test strip must be inserted into a meter, thereby making electrical contact with somewhat rigid conductive contacts. During insertion and removal of the test strip, the electrical contacts may scratch or abrade material off the surface of the test strip. This can cause a number of problems. For example, abraded material could theoretically build up within the test meter, and in some cases, may hypothetically interfere with electrical contacts in subsequent tests, thereby producing erroneous test results, or even causing complete measurement failure. In addition, abrasion of test strip surfaces could possibly disrupt a test strip autocalibration system or cause faulty electrical contacts, again leading to erroneous or faulty meter operation. Therefore, the present inventors have identified a need for test strips that are resistant to abrasion by meter contacts. Document WO 2005/088319 describes a diagnostic test strip according to the preamble of claim 1.

### SUMMARY OF THE INVENTION

Embodiments of the present invention are directed to a diagnostic test strip, and a method of making a plurality of test strips that obviate one or more of the limitations and disadvantages of prior devices and methods.

One embodiment of the invention is directed to a diagnostic test strip as defined in claim 1.

A second embodiment of the invention is directed to a method of producing a diagnostic test strip as defined in claim 4.

Additional objects and advantages of the invention will be set forth in part in the description which follows, and in part will be obvious from the description, or may be learned by practice of the invention. The objects and advantages of the invention will be realized and attained by means of the elements and combinations particularly pointed out in the appended claims.

It is to be understood that both the foregoing general description and the following detailed description are exemplary and explanatory only and are not restrictive of the invention, as claimed.

### BRIEF DESCRIPTION OF THE DRAWINGS

FIG. 1 is a view of a test strip according to an embodiment of the present invention.
FIG. 2 is a top perspective view of a test strip inserted within a meter strip connector according to an embodiment of the present invention.
FIG. 3 is a cross-sectional view of a test strip inserted within a meter strip connector according to an embodiment of the present invention.
FIG. 4A is a top view of a distal portion of a test strip illustrating breaks dividing particular regions of the test strip connecting end according to an embodiment of the present invention.
FIG. 4B is a top view of a distal portion of a test strip illustrating conductive regions forming electrical contacts according to an embodiment of the present invention.
FIG. 4C is a top view of a distal portion of a test strip illustrating a particular arrangement for a plurality of electrical contacts according to an embodiment of the present invention.
FIG. 4D is a top view of a distal portion of a test strip illustrating multiple insulators covering particular regions of the test strip connecting end according to an embodiment of the present invention.
FIG. 5 is an expanded top view of a distal portion of a test strip inserted within a meter strip connector according to an embodiment of the present invention.
FIG. 6 is a top view of a distal portion of a test strip illustrating a plurality of electrical contacts forming a code.
FIG. 7 is a simplified schematic diagram of the electrical connections between a meter and a plurality of electrical contacts of a test strip according to an embodiment of the invention.
FIG. 8 is an alternative simplified schematic diagram of the electrical connections between a meter and a plurality of electrical contacts of a test strip according to an embodiment of the invention.
FIG. 9 is a cross-sectional view of a distal section of a test strip, which may form an electrical connection with a test meter, according to an exemplary disclosed embodiment.

### DESCRIPTION OF THE EMBODIMENTS

Reference will now be made in detail to the exemplary embodiments of the invention, examples of which are illustrated in the accompanying drawings. Wherever possible, the same reference numbers will be used throughout the drawings to refer to the same or like parts.

An individual test strip may also include an embedded code relating to data associated with a lot of test strips, or data particular to that individual strip. The embedded information presents data readable by the meter signaling the meter's microprocessor to access and utilize a specific set of stored calibration parameters particular to test strips from a manufacturing lot to which the individual strip belongs, or to an individual test strip. The system may also include a check strip that the user may insert into the meter to check that the instrument is electrically calibrated and functioning properly.

In addition, the present disclosure provides test strips that include electrical contacts that are resistant to scratching or abrasion. In other embodiments, the test strips of the present disclosure can include electrical contacts having material properties and dimensions such that, even when scratched or abraded, the test strips will continue to function properly. Such test strips include conductive electrical contacts formed of two or more layers of material. A first lower layer includes a conductive metal, ink, or paste. A second upper layer includes a conductive ink or paste. Further, in some embodiments, the upper layer can have a resistance to abrasion that is greater than the lower layer. In addition, the second upper layer may have a thickness such that, even when scratched or abraded, the entire thickness of the conductive layer will not be removed, and the electrical contact will continue to function properly.

For purposes of this disclosure, "distal" refers to the portion of a test strip further from the fluid source (i.e. closer to the meter) during normal use, and "proximal" refers to the portion closer to the fluid source (e.g. a finger tip with a drop of blood for a glucose test strip) during normal use.

The test strip may include a sample chamber for receiving a user's fluid sample, such as, for example, a blood sample. The sample chamber and test strip of the present specification can be formed using materials and methods described in commonly owned U.S. Patent No. 6,743,653. Accordingly, the sample chamber may include a first opening in the proximal end of the test strip and a second opening for venting the sample chamber. The sample chamber may be dimensioned so as to be able to draw the blood sample in through the first opening and to hold the blood sample in the sample chamber by capillary action. The test strip can include a tapered section that is narrowest at the proximal end, or can include other indicia in order to make it easier for the user to locate the first opening and apply the blood sample.

It should also be noted that although the test strip is shown as an elongated, approximately rectangular structure, the test strip can also include other shapes. For example, the test strip can also include tabs, discs, or any other suitable configuration, and although such configurations may not traditionally be considered 'strips,' it is understood that 'test strip,' as used herein, is understood to encompass any test media configuration suitable for use with a meter that makes electrical contact with a sample collection device (i.e. a test strip).

A working electrode and counter electrode can be disposed in the sample chamber optionally along with fill-detect electrodes. A reagent layer is disposed in the sample chamber and preferably contacts at least the working electrode. The reagent layer may include an enzyme, such as glucose oxidase or glucose dehydrogenase, and a mediator, such as potassium ferricyanide or ruthenium hexamine. The test strip has, near its distal end, a first plurality of electrical strip contacts that are electrically connected to the electrodes via conductive traces. In addition, the test strip may also include a second plurality of electrical strip contacts near the distal end of the strip. The second plurality of electrical contacts can be arranged such that they provide, when the strip is inserted into the meter, a distinctly discernable lot code readable by the meter. As noted above, the readable code can be read as a signal to access data, such as calibration coefficients, from an on-board memory unit in the meter.

In order to save power, the meter may be battery powered and may stay in a low-power sleep mode when not in use. When the test strip is inserted into the meter, the first and second plurality of electrical contacts on the test strip form electrical connections with corresponding electrical contacts in the meter. The second plurality of electrical contacts may bridge a pair of electrical contacts in the meter, causing a current to flow through a portion of the second plurality of electrical contacts. The current flow through the second plurality of electrical contacts causes the meter to wake up and enter an active mode. The meter also reads the code information provided by the second plurality and can then identify, for example, the particular test to be performed or a confirmation of proper operating status. In addition, based on the particular code information, the meter can also identify the inserted strip as either a test strip or a check strip. If the meter detects a check strip, it performs a check strip sequence. If the meter detects a test strip, it performs a test strip sequence.

In the test strip sequence, the meter validates the working electrode, counter electrode, and, if included, the fill-detect electrodes, by confirming that there are no low-impedance paths between any of these electrodes. If the electrodes are valid, the meter indicates to the user that a sample may be applied to the test strip. The meter then applies a drop-detect voltage between the working and counter electrodes and detects a fluid sample, such as, a blood sample, by detecting a current flow between the working and counter electrodes (i.e., a current flow through the blood sample as it bridges the working and counter electrodes). To detect that an adequate sample is present in the sample chamber and that the blood sample has traversed the reagent layer and mixed with the chemical constituents in the reagent layer, the meter may apply a fill-detect voltage between the fill-detect electrodes and measure any resulting current flowing between the fill-detect electrodes. If this resulting current reaches a sufficient level within a predetermined period of time, the meter indicates to the user that adequate sample is present and has mixed with the reagent layer.

The meter can be programmed to wait for a predetermined period of time after initially detecting the blood sample to allow the blood sample to react with the reagent layer. Alternatively, the meter may be configured to immediately begin taking readings in sequence. During a fluid measurement period, the meter applies an assay voltage between the working and counter electrodes and takes one or more measurements of the resulting current flowing between the working and counter electrodes. The assay voltage is near the redox potential of the chemistry in the reagent layer, and the resulting current is related to the concentration of the particular constituent measured, such as, for example, the glucose level in a blood sample.

In one example, the reagent layer may react with glucose in the blood sample in order to determine the particular glucose concentration. In one example, glucose oxidase or glucose dehydrogenase is used in the reagent layer. During a sample test, the glucose oxidase initiates a reaction that oxidizes the glucose to gluconic acid and reduces a mediator such as ferricyanide or ruthenium hexamine. When an appropriate voltage is applied to a working electrode relative to a counter electrode, the ferrocyanide is oxidized to ferricyanide, thereby generating a current that is related to the glucose concentration in the blood sample. The meter then calculates the glucose level based on the measured current and calibration data that the meter has been signaled to access by the code data read from the second plurality of electrical contacts associated with the test strip. The meter then displays the calculated glucose level to the user. Each of the above-described components and their interconnection will now be described.

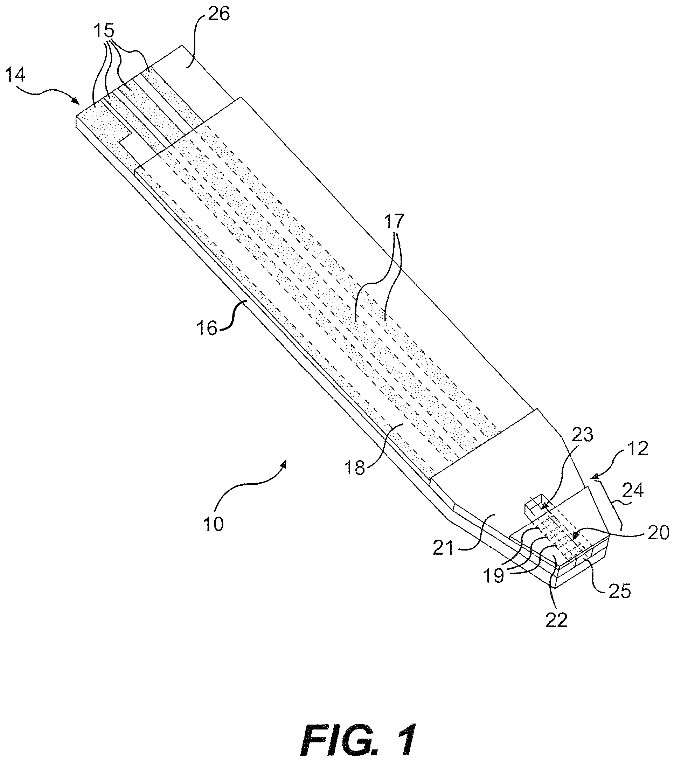

FIG. 1 illustrates a cross-sectional view of an embodiment of a test strip 10. Test strip 10 includes a proximal connecting end 12, a distal end 14, and a base layer 16 extending along the entire length of test strip 10. Base layer 16 is preferably composed of an electrically insulating material and has a thickness sufficient to provide structural support to test strip 10. For purposes of this application, an insulating material (e.g. an insulating layer, coating, ink, or substrate etc.) comprises any material in which electrons or ions cannot be moved easily, hence preventing the flow of electric current. Accordingly, an element can be said to be insulated when it is separated from other conducting surfaces by a dielectric substance or air space permanently offering a high resistance to the passage of current and to disruptive discharge through the substance or space. By contrast, for purposes of this application, a resistive element, is one that introduces an increased level of impedance into a circuit that reduces (but does not necessarily prevent) the flow of electric current. Base layer 16, for example, may be polyester that is about 0.254 to about 0.355 mm (about 0.01 to about 0.014 inches) thick. although other sizes may be used depending on the particular application and manufacturing method. Disposed on base layer 16 is a conductive pattern.

The conductive pattern includes a plurality of electrodes 19 disposed on base layer 16 near proximal end 12, a plurality of electrical strip contacts 15 disposed on base layer 16 near distal end 14, and a plurality of conductive traces 17 electrically connecting the electrodes to the plurality of electrical strip contacts. For purposes of this application, the noun "contact" denotes an area intended for mechanical engagement with another corresponding "contact" irrespective of whether an electric circuit is completed or passes through the particular area.

In one embodiment, the plurality of electrodes 19 may include a working electrode, a counter electrode, and fill-detect electrodes. The conductive pattern is produced by applying a conductive material to base layer 16. The electrode material may be provided by thin-film vacuum sputtering of a conductive material (e.g. gold) and/or a semiconductive material (e.g. indium-zinc oxide) onto the base layer 16. The resulting electrode layer can then by further patterned according to the specific application by forming particular conductive regions/pathways through a laser ablation process. Alternative materials and methods for providing a conductive pattern, in addition to screen printing, can be employed without departing from the scope of the invention.

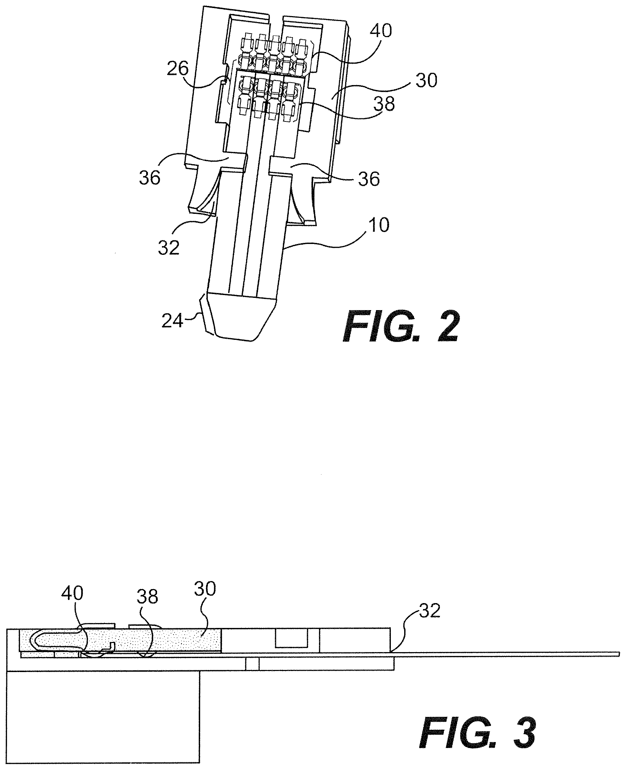

In order to prevent scratching and other damage to the conductive traces 17, a dielectric insulating layer 18 can be formed over the conductive pattern along a portion of the test strip between the measuring electrodes and the plurality of electrical strip contacts. As seen in FIG. 1, the proximal end 12 of test strip 10 includes a sample receiving location, such as a sample chamber 20 configured to receive a patient's fluid sample, as described above. The sample chamber 20 may be formed in part through a slot formed between a cover 22 and the underlying measuring electrodes formed on the base layer 16. Further, in some embodiments, a spacer material 21 may be disposed on base 16, and the sample chamber 20 may be formed within the spacer material 21. In some embodiments, dielectric layer 18 and spacer 21 may be formed of a single piece of material. In addition, the sample chamber 20 may include a vent hole 23 to allow gases contained within sample chamber 20 to be vented as a sample enters through a proximal opening 25. The relative position of the measuring electrodes and the electrical strip contacts form a proximal electrode region 24 at one end of strip 10 and a distal strip contact region 26 at the other end, as shown in FIG. 2.

Referring to FIG. 2, a top perspective view of a test strip 10 inserted within a meter connector 30 is illustrated. As seen in FIG. 2, the strip 10 includes a proximal electrode region 24, which contains the sample chamber and measuring electrodes described above. The proximal electrode region 24 may have a particular shape in order to aid a user in distinguishing the end receiving a fluid sample from the distal strip contact region 26. The meter connector 30 includes channel 32 extending out to a flared opening for receiving the test strip 10. The connector 30 may further include tangs 36 extending a predetermined height above the base of channel 32. The predetermined height of tangs 36 is selected to limit the extent to which a test strip 10 can be inserted into channel 32, such as through a corresponding raised layer of test strip 10.

FIG. 3 provides a cross-sectional view of a test strip inserted within a meter strip connector 30 is illustrated. The connector 30 further includes a first plurality of connector contacts 38, disposed closer to the proximal end of the connector 30 and a second plurality of connector contacts 40 disposed closer to the distal end of the connector 30. As illustrated, the test strip 10 is inserted into the flared opening with the distal strip contact region 26 extending first through the connector channel 32.

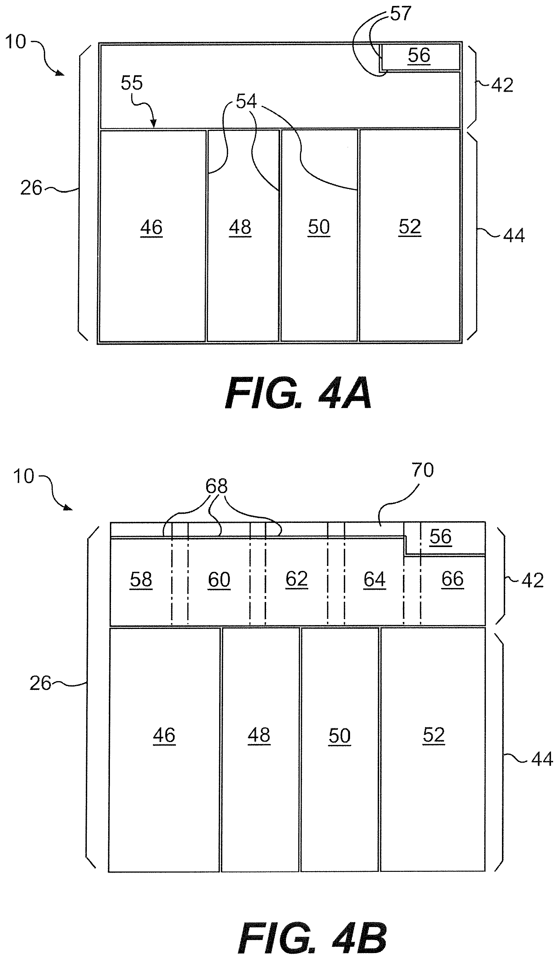

FIG. 4A is a top view of a distal portion of a test strip 10 illustrating the distal strip contact region 26. The conductive pattern formed on base layer 16 extends along strip 10 to include the distal strip contact region 26. As illustrated in FIG. 4A, distal strip contact region 26 is divided to form two distinct conductive regions, 42 and 44 respectively. Conductive region 44 is divided into four columns forming a first plurality of electrical strip contacts, labeled 46, 48, 50, and 52. The first plurality of electrical strip contacts are electrically connected to the plurality of measuring electrodes at the distal end of the test strip 10 as explained above. It should be understood that the four contacts 46-52 are merely exemplary, and the system could include fewer or more electrical strip contacts corresponding to the number of measuring electrodes included in the system.

The first plurality of electrical strip contacts 46-52 are divided, for example, through breaks 54 formed through the underlying conductive pattern in the test strip 10. These breaks could be formed in the conductive pattern during printing, through a scribe process, laser ablation, or through a chemical/ photo-etching type process. In addition, other processes of forming conductive breaks by removing a conductor in the test strip 10 may be used as would be apparent to one having ordinary skill in the art. An additional break 55 divides conductive region 44 from conductive region 42 within distal strip contact region 26, and a further break 57 separates the upper right-hand portion of distal strip contact region 26 to form a notch region 56, as will be described more fully in detail below.

FIG. 4B illustrates an additional view of the distal strip contact region 26. In FIG. 4B, conductive region 42, described above with regard to FIG. 4A, is divided into five distinct regions outlining a second plurality of electrical strip contacts forming contacting pads 58, 60, 62, 64, and 66. The second plurality of electrical strip contacts forming contacting pads 58, 60, 62, 64, and 66, can be divided through the same process used to divide the first plurality of electrical strip contacts, 46, 48, 50, and 52, described above. The contacting pads 58, 60, 62, 64, and 66 are configured to be operatively connected to the second plurality of connector contacts 40 within meter connector 30. Through this operative connection, the meter is presented with, and reads from the contacting pads, a particular code representing information signaling the meter to access data related to the underlying test strip 10. In addition, FIG. 4B depicts a further pattern of breaks 68, isolating an outermost distal connecting end 70 of the distal strip contact region 26.

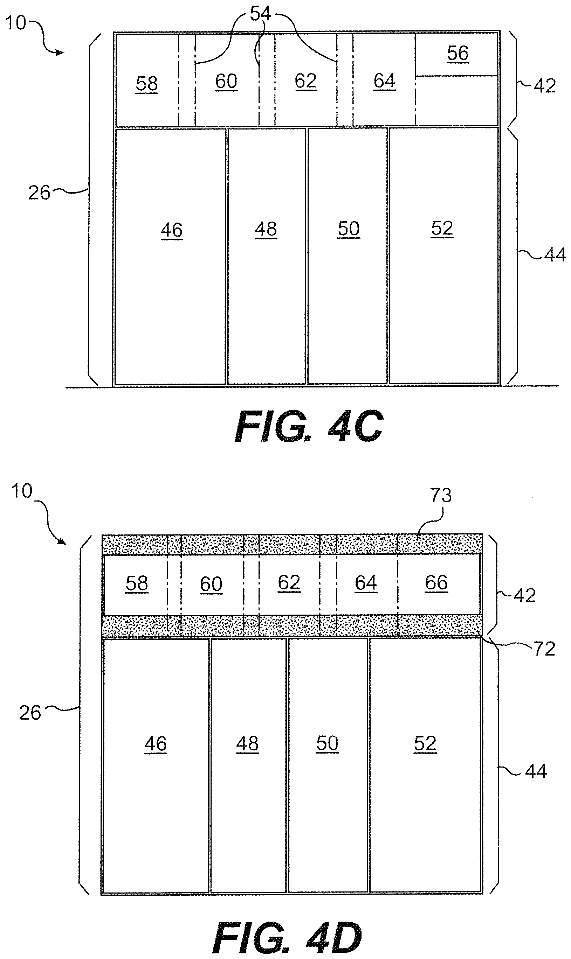

FIG. 4C illustrates an additional view of the distal strip contact region 26. In FIG. 4C, the distal strip contact region 26 is depicted to include the first plurality of electrical strip contacts 46-52, the second plurality of electrical strip contacts forming contacting pads 58, 60, 62, 64, 66, and the separated notch region 56. As noted, the above described conductive regions can all be formed as a result of breaks 54 within the underlying conductive pattern of test strip 10.

FIG. 4D illustrates additional features of the distal strip contact region 26. A strip of non-conductive insulating ink 72 can provide further separation between conductive region 44 and conductive region 42 within distal strip contact region 26. The borders between the two regions can be printed with the insulating ink 72 in order to maintain distinct areas of conductivity (bordered by a distinct area of insulation) and to prevent scratching by meter connector contacts during the strip insertion process, which can adversely affect the desired conductivity of one of the strip contacts. The non-conductive insulating ink 72 can be administered, for example, through a screen printing process. Such screen printing of a dielectric insulating coating is advantageous in that it can be applied later in the strip manufacturing process and in an easily programmable/reproducible pattern. The additional step of adding such an insulating coating can be less expensive and time consuming than methods requiring substrate ablation. For example, ablating a substrate surface through a laser or chemical ablation process involves a time consuming process of precisely removing a particular pattern of preexisting material.

FIG. 4D illustrates that test strip 10 may include another strip of non-conductive insulating ink 73 formed at the distal end of the test strip 10. The strip of non-conductive insulating ink 73 provides a non-conductive region at the distal end of the strip 10. The strip 73 thereby prevents any meter connector contacts from creating an active conductive connection with any portion of contacting pads 58, 60, 62, 64, and 66 before the strip is fully inserted into the meter. Accordingly, strip 73 provides an additional feature for assuring a proper connection between the test strip 10 and the corresponding meter.

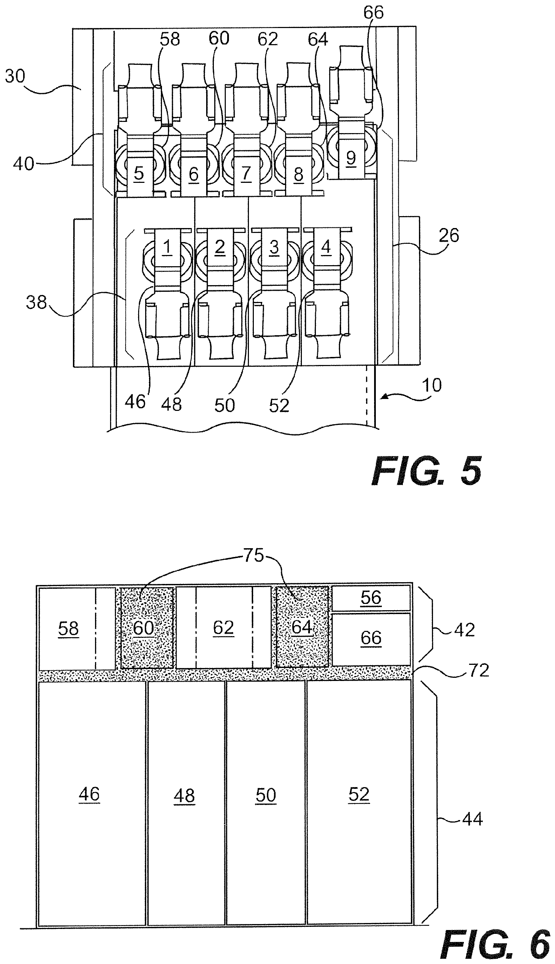

Referring to FIG. 5, meter strip connector 30 is illustrated receiving a distal strip contact region 26 of test strip 10. FIG. 5 depicts a first plurality of connector contacts 38, labeled 1-4 respectively, and a second plurality of connector contacts 40, labeled 5-9. The connector contacts 38 and 40 make contact with distinct portions of the distal strip contact region 26. In particular, upon proper insertion of the test strip 10 into connector 30, the electrical strip contacts 46-52, which form the first plurality of electrical strip contacts, are respectively electrically connected to the connector contacts 1-4, which form the first plurality of connector contacts 38. Similarly, the contacting pads 58, 60, 62, 64, and 66, which form the second plurality of electrical strip contacts, are respectively electrically connected to the connector contacts 5-9, which form the second plurality of connector contacts 40.

As seen in FIG. 5, the first plurality of connector contacts 38 are laterally staggered or offset relative to the second plurality of connector contacts 40. Although the first and second plurality are illustrated as being in distinct rows and offset from each other, they need not be in distinct rows and can instead be offset in an additional manner, such as, for example, in distinct groups. Accordingly, as a test strip 10 is inserted into meter connector 30, the conductive signal provided by contacting pads 58-66 is unhindered by any scratches or scuffs that would otherwise result from first sliding contacting pads 58-66 under connector contacts 1-4 in order to reach their destination connection at connector contacts 5-9. Therefore, the staggered arrangement of connector contacts 38 relative to connector contacts 40 provides a more reliable connection. In addition, the application of strip 72 of non-conductive insulating ink (FIG. 4D) also assists in preventing the conductive coating from one of contacting pads 58-66 from being scratched and "plowed" away by the friction and interaction from the meter connector contacts 38. Accordingly, strip 72 of non-conductive insulating ink provides increased reliability of connector and contact conduction.

In one embodiment, the connection between contacting pad 66 and connector contact 9 establishes a common connection to ground (or a voltage source where the polarity is reversed), thereby completing an electric circuit, which includes the meter and at least a portion of conductive region 42. The completion of this circuit can perform a meter wake-up function, providing a signal to the meter to power up from low-power sleep mode. Therefore, as illustrated in FIG. 5, the connector contact 9 may be positioned proximally relative to the remaining contacts 5-8, in order to ensure that connectors 5-8 are in proper connecting position prior to the final closing/wake-up of the circuit through the connection of contacting pad 66 and connector contact 9. Furthermore, because the non-conductive insulating ink strip 73 (See FIG. 4D) can be formed at the distal end of the test strip 10 and also because a conducting substance can be removed from notch region 56 (See FIG. 4C), premature wake-up of the meter will be prevented.

In other words, during distal movement of test strip 10 within the connector channel 32, the common connection will not be established at the point connector contact 9 engages the extreme distal edge of test strip 10. Instead, common connection will be established only when the connector contact passes notch 56, and ink strip 73, if applied, and engages a conductive portion of contacting pad 66. Accordingly, the combination of a proximally positioned connector contact 9 and a non-conductive notch region 56 provides a more reliable connection between strip 10 and the meter.

As noted above, the contacting pads 58, 60, 62, 64, and 66 are configured to be operatively connected to the second plurality of connector contacts 40 within meter connector 30. Through this operative connection, the meter is presented with, and reads from the contacting pads, a particular code signaling the meter to access information related to a particular underlying test strip 10. The coded information may signal the meter to access data including, but not limited to, parameters indicating the particular test to be performed, parameters indicating connection to a test probe, parameters indicating connection to a check strip, calibration coefficients, temperature correction coefficients, pH level correction coefficients, hematocrit correction data, and data for recognizing a particular test strip brand.

One such code is illustrated in FIG. 6, where conductive contacting pads 60 and 64 are overprinted with an electrical insulting material, such as, for example, a non-conductive (insulating) ink layer 75. A non-conductive ink layer 75 significantly increases the impedance (and may even preventing the flow of electric current there through) between the corresponding connector contacts (in this example, connector contacts 6 and 8) and the underlying strip portion at various predetermined contacting pads within the conductive region 42 of distal strip contact region 26. As described above with regard to FIG. 4D, the use of non-conductive insulating ink 75 may be desirable for other methods of altering the conductivity of a strip portion.

An exemplary insulating material includes, but is not limited to, VISTASPEC HB Black, HB Yellow, HB Cyan, and BrightWhite HB available from Aellora™ Digital of Keene, New Hampshire. The VISTASPEC HB and BrightWhite HB materials are hybrid UV-curable inks for use in elevated temperature piezo drop-on-demand ink jet arrays. This VISTASPEC ink is jetted at an elevated temperature, rapidly sets upon contact with the underlying substrate, and is then cured by UV radiation. The ink's properties include electrical insulation, resistance to abrasion from a meter's contacts, enhanced adhesion to an underlying conductive material, and beneficial visco-elastic characteristics. The material's visco-elastic characteristics minimize ink spreading on the underlying substrate. Furthermore, these visco-elastic characteristics enable this ink to be utilized with high print resolution piezo technology that enables accurate and precise patterning of the VISTASPEC ink onto the conductive electrode substrate. In addition, the visco-elastic characteristics of the VISTASPEC ink enables a sample as small as about an 80 picoliter drop to remain pinned at the location where it makes contact with the underlying substrate, thereby enabling precise pad sizes, positional accuracy, and precision of up to less than about 0.127 mm (0.005 inches). As an example, printing of the insulating material can be accomplished through the use of a SureFire Model PE-600-10 single pass piezo drop-on-demand ink jet print engine, also available from Aellora™ Digital of Keene, New Hampshire. As non-limiting examples, the above described ink jet print engine can utilize Nova and Galaxy model print heads available from Spectra Inc. of Lebanon, New Hampshire.

Systems using a laser or chemical ablation process require significant time to precisely remove a particular pattern of preexisting material. Because coding of the strip occurs later in the assembly process than the ablation step, adding a non-conductive ink layer 75 to the contacting pads eliminates the tolerance issues that would result from reintroducing strips into a larger ablation process for coding. Such printing of a dielectric insulation coating is advantageous in that it can be applied later in the strip manufacturing process and in an easily programmable and/or reproducible pattern. As a non-limiting example, the method of providing layer 75 to the underlying substrate can include the use of at least one registration datum along the underlying strip to insure accurate formation of the layer 75 according to a particular desired pattern. For example, datums can be provided orthogonally (e.g. longitudinally and laterally) along a substrate where that can be mechanically or optically referenced by a printing apparatus to facilitate the formation of an accurate and reproducible pattern.

Upon connection of the contacting pads 58, 60, 62, 64, and 66 in FIG. 6 to the corresponding connector contacts 40, the meter will read a particular code based on the number and pattern of contacting pads overprinted with a non-conductive ink layer 75. In other words, the use of non-conductive ink layer 75 provides a switching network to be read by the meter. When an insulator is printed over one of the conductive surfaces of contacting pads 58, 60, 62, 64, and 66, it prevents the flow of electric current therealong and alters the conductive path between the contacting pad and connector contact (e.g. where no current flows). When no insulator is printed over the conductor current flow is relatively unimpeded (a low impedance path).

Upon reading a particular code, an internal memory within the meter can access, through a stored microprocessor algorithm, specific calibration information (such as, for example, calibration coefficients) relating to the particular test strip. The meter can read the code through either an analog or digital method. In the analog mode, a preset resistive ladder is interconnected within the meter to the second plurality of connector contacts 40 (labeled 5-9 in FIG. 5) such that permutations of printed non-conductive ink can be correlated to a distinct lot code using a voltage drop, resistance, or current measurement. The analog method also can be simultaneously used as the auto-on/wake-up feature as long as each code has at least one pad free of non-conductive ink that can make a low impedance connection to wake the meter up by closing an open circuit. The analog voltage, resistance, or current level could be used to signal the meter to access any of the data referenced above particular to the underlying test strip.

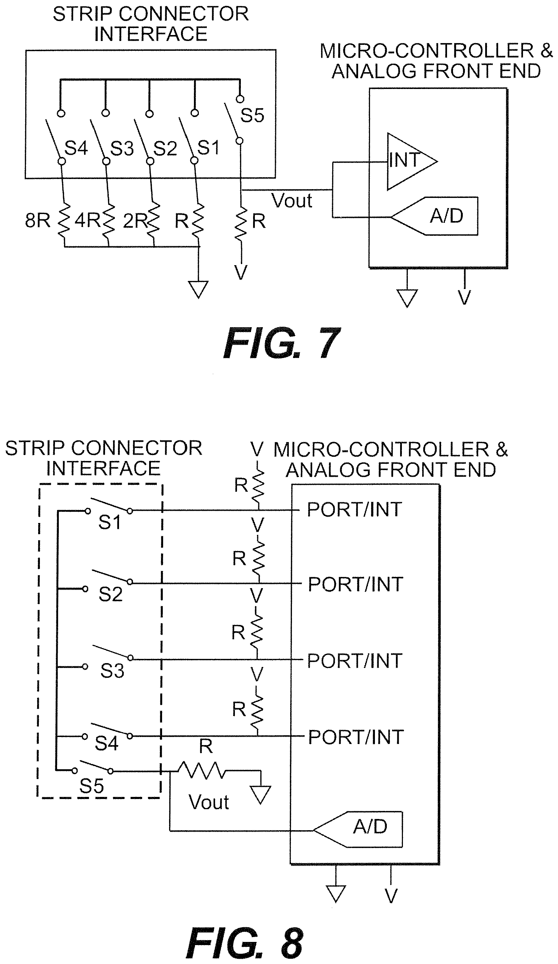

FIG. 7 depicts a schematic diagram of the electrical connections between a meter and contacting pads 58, 60, 62, 64, and 66 of a test strip, according to an embodiment of the invention. Switch S5 of FIG. 7 provides the connection to a single voltage source V. Accordingly, switch S5, represents the required connection of contacting pad 66 and connector contact 9 in the analog code reading process. Switches S4-S1 schematically represent the connection between connector contacts 5-8 and contacting pads 58-64 of FIG. 5, respectively. When a non-conductive ink layer 75 is provided over one of the contacting pads 58, 60, 62, and 64, the corresponding switch, S4, S3, S2, or S1, will prevent the flow of electric current therealong upon physical engagement with corresponding connector contacts 5-8. Accordingly, a particular code will correspond to a particular switching configuration, in the switch network of FIG. 7.

As further seen in FIG. 7, each of switches S4-S1 close to add a distinct value of additional impedance to the closed circuit by bridging the connection to a particular resistor. Therefore, through the application of Ohm's and Kirchhoff's laws, a circuit measurement at Vₒᵤₜ will provide distinct values based on the particular code presented by test strip 10. In an alternative embodiment, the direction of current flow can be reversed, if desired, by connecting switch S5 to common ground and instead connecting the resistor R to the single voltage source.

In the digital mode, as schematically represented in FIG. 8, each contacting pad 58-66, would be read as an individual input, unlike the single input used by the analog method. For the digital method to be simultaneously used as an auto-on/wake-up feature, the inputs would need to be wired together or connected to an interrupt controller of a micro-controller. Each code must have at least one pad free of non-conductive ink 75 such that a low impedance connection can be made to wake-up the meter's micro-controller.

Pads including non-conductive ink 75 with levels of high and low impedance produce a binary code yielding a code index based on the number of pads (P) implemented, where the number of codes is N = 2^{P}. The number of codes possible when integrated with an auto-on/wake-up feature is reduced to N = 2^{P}-1. Accordingly, a code with all zeros (all insulators) is not an active code as it will not wake up the meter.

When a strip 10 is inserted into the meter connector 30, one contact is closed and wakes up the meter by pulling the microcontroller's interrupt either high or low. The meter will then check the voltage out (Vₒᵤₜ) to determine the test type and read the code bits (S1, S2, S3, S4) to determine the code value. The code value can, for example, be associated with a stored set of coefficients in the meter's memory for use in a glucose-mapping algorithm that is particularly correlated to the reagent applied to the measuring electrode region. This code can also be associated with other types of strip parameter information, such as those referenced above. It could also select different meter configuration options as well. The voltage drop across the series resistor R at Vout in FIG. 8 can be sensed to determine if code values are within a predetermined range for use as a confirmation signal. This can also be used to for strip identification (check strip, manufacturing probe, and different test type).

In addition to providing either a high or low impedance level through the application or absence of an insulating layer of non-conductive ink 75 over one of the contacting pads, a particular resistive element may be applied over a particular contacting pad. The resistive element introduces an increased level of impedance into a circuit that reduces, but does not necessarily prevent, the flow of electric current. Accordingly, the use of a specific resistive element over a particular contacting pad provides an intermediate level of resistance to the contacting pad of the test strip. When this intermediate level of resistance is connected to the meter through engagement with a corresponding meter connector contact, the meter can detect this "intermediate" level (e.g. through a circuit measurement of voltage drop by applying Ohm's and Kirchhoff's laws).

The detection of such an intermediate level can alert the meter's processor to access a new set of code data relating to the particular test strip. In other words, a resistive element coating can be used to expand the number of codes available with a set number of contacting pads. For example, a strip may be formed with a particular code through a particular pattern of non-conducting insulating ink 75. When one of the conducting contact pads is formed to include a particular resistive element, that same code represented by the pattern of non-conducting ink 75 now can be read by the meter to access an entirely different set of data.

As an example, the contacting pad 66 of FIG. 6 (or any of the available contacting pads) could be formed to include a resistive element. As a non-limiting example, the resistive element could be provided in the form of a printed conductive ink. The thickness of the printed ink forming the resistive element, as well as the resistivity of the ink composition, can be varied to achieve the desired resistance for a particular contacting pad. The additional information made available through this expansion of codes can include, but is not limited to, information related to hematocrit correction, information related to meter upgrades, and information related to the particular strip type. Accordingly, the use of such a resistive element can be used to expand the number of code configurations available with a set number of contacting pads.

It should be noted that the particular disclosed configurations of test strip 10, including the configuration of connector contacts 38, 40 and the corresponding first and second plurality of electrical strip contacts, are merely exemplary, and different configurations could be formed without departing from the scope of the invention. For example, the underside of strip 10 can be formed to incorporate an additional number of contacting pads in order to increase the size (and thereby the amount of information) in the code index. The additional contacting pads on the underside of strip 10 could represent a third plurality of electrical strip contacts, thereby increasing the number of codes available.

The incorporation of individualized code data within individual test strips provides numerous advantages in addition to those associated with accuracy of measurement. For example, with individual strip coding, a user no longer needs to manually enter the meter's lot code, thereby removing the possibility of user error for this step. Strip lot codes stored directly on individual test strips will also provide a means to ship mixed lots of strips in a single strip vial. In contrast, current technologies such as button/key coding require all strips (typically packaged in a vial including 50 strips from the same lot) in a vial to be from the same lot code.

Individual strip coatings also afford bulk packaging benefits. For example, mixed lot test strips and vials including different numbers of strips will be possible. Strips from various lots could be stored in a central location and packaged for sale without the added time and expense required to provide strips that are packaged from a single lot. Individual lot calibration codes stored on strips can also provide a means for varying a code across a single lot should a strip lot have variation from beginning to end, or anywhere in between. Predetermined variations in manufacturing within a strip lot can be corrected by applying a continuously changing code across the lot, thereby solving yield problems and improving in-lot strip-to-strip variation. In addition, embedding lot codes on individual strips can be used to distinguish different types of test strips (e.g. glucose vs. ketone), identify check strips, or identify different manufacturing procedures, provide data for meter upgrades, and to correlate particular test strips for use only with a specific meter or meter type.

As noted previously, in some embodiments, the test strips of the present invention can include a conductive pattern including at least one electrical strip contact. The electrical strip contacts may be disposed at the distal end 14 of a test strip 10, such that the electrical strip contacts may form electrical contacts with connector contacts 38, 40 of a test meter. A first conductive material is applied to the base layer 16 to form a conductive pattern. Subsequently, a second conductive material is applied over the first conductive material. Further, in some embodiments, the second conductive material may be selected such that the second conductive material has a higher resistance to abrasion than the first conductive material.

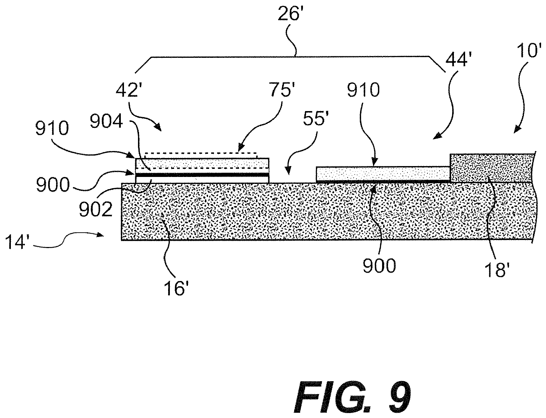

FIG. 9 is a cross-sectional view of a distal section 14' of a test strip 10', which may form an electrical connection with a test meter, according to an exemplary disclosed embodiment. As shown, test strip 10' includes a base layer 16' and a distal strip contact region 26'. Distal strip contact region 26' can include one or more groups of conductive contacts 42', 44'. However, in some embodiments, a single contact region may be used, or more than two contact regions may be produced. Further, each contact region may include multiple contacts, as described previously with respect to FIGS. 4-6. As shown, contacts 42', 44' are separated by a break 55'. The break may include an open space, as produced by a laser ablation or chemical/photo etching process. Alternatively, break 55' may be filled with an insulating material, as described previously.

As shown, contacts 42', 44' can include two layers 900, 910. In some embodiments, first layer 900 may be disposed directly on base layer 16', and second layer 910 may be applied on top of first layer 900. Further, first layer 900 includes a conductive metallic material, such as gold, titanium, palladium, silver, platinum, copper, or any other suitable metallic conductor. Alternatively, first layer 900 may include a conductive material, including, for example, copper pastes/inks, silver paste/inks, gold pastes/inks, palladium pastes/inks, and/or any other suitable paste or ink. In addition, second layer 910 includes a carbon-based material (e.g. carbon/graphite paste), and/or any other suitable paste or ink.

It should also be noted that one or more semiconductive layers may be included. For example, it may be desirable to include a semiconductive layer 902 below the first layer 900 and in contact with base 16. Alternatively, a semiconductive layer 904 may be placed on top of first layer 900, and between first layer 900 and second layer 910. Suitable semiconductive materials may include, for example, indium-zinc oxide. The specific semiconductive materials may be selected based on desired electrical properties, and/or their ability to adhere to the base 16, first layer 900, and/or second layer 910. Further, in some embodiments, first layer 900 may include only a semiconductive material having sufficient thickness to provide adequate electrical conduction.

In addition, in some embodiments, first layer 900 may include a conductive layer, including for example, a metallic conductor such as gold. In addition, an adhesion layer may be placed between a metallic gold layer 900 and substrate base 16, at a position consistent with layer 902, as shown in FIG. 9. The adhesion layer may include a metallic or semiconductive material, such as, for example titanium.

As noted previously, the first layer 900 can be produced using a variety of suitable deposition processes. After the first layer 900 is produced, the second layer 910 may be applied on top of the first layer 900. In some embodiments, the contact patterns, including first layer 900 and second layer 910, may be produced by collectively forming breaks in both first and second layers 900, 910, either simultaneously, or sequentially. As described previously, such breaks may be formed using various scribing processes, laser ablation, and/or through chemical/photo-etching processes. A number of suitable laser ablation processes may be used to produce a desired pattern for the electrical contacts. For example, one suitable laser ablation system includes a Nd:YVO₄ Prisma 1064-32-V laser by Coherent. However, any suitable laser may be selected to produce desired material dimensions and patterns.

The second layer may be produced using a variety of suitable deposition processes. The specific process may be selected based on cost, desired feature dimensions, and/or the specific materials selected for the second layer 910. In some embodiments, the second layer 910 may be produced using a screen-printing process, a gravure printing process, an ink-jet process, a spray printing process, and/or flexographic printing processes. For example, suitable materials can include various conductive pastes and/or inks. Suitable pastes materials can include various conductive pastes and/or inks. Suitable pastes and/or inks can include, for example, carbon/graphite paste (Gwent Electronic Materials Ltd, C2000802D2), water-based carbon ink (Acheson, PE-003), conductive graphite coating (Acheson, SS 24600), carbon conductive composition (DuPont, 5067), carbon conductor paste (DuPont, 5085), silver/carbon conductor paste (DuPont, 5524),

In some embodiments, the selected application process may be based on the desired layer thickness, and/or material type. For example, suitable processes for depositing layers of conductive inks with thicknesses ranging from about 1 micron to about 50 microns can include gravure printing, ink jet printing, spray deposition. For materials from about flexography for thicknesses of about 1 micron to about 15 microns, flexography may be selected. For thicker films (e.g. about 10 microns to about 50 microns) screen printing processes may be employed. Printed inks may be cured using ovens, IR heat sources, or UV lamps for about 5 seconds to about 5 min depending on ink formulation requirements.

Suitable materials for the second layer 910 may be selected to provide increased resistance to abrasion by electrical contacts 38, 40. Abrasion by contacts 38, 40 can disrupt electrical connections with a meter and also alter the calibration data provided by electrical contact patterns. In addition, material abraded by contacts 38, 40 may collect within a test meter connection region, potentially disrupting future tests. Therefore, in some embodiments, the second layer 910 may be produced with a thickness sufficient to prevent abrasion through second layer 910 and/or first layer 900. A range of suitable thicknesses may be included for first layer 900 and/or second layer 910. For example, first layer 900, (with or without one or more semiconductive layers 902, 904) may be between about 1 and about 50 microns thick. Further, second layer 910 may be between about 10 microns and about 60 microns. In addition, in some embodiments, second layer 910 may be thicker than first layer 900.

In some embodiments, as noted previously, one or more regions of insulative material 75' may be applied either over conductive region 42', 44'. The insulative material may be applied in a pattern corresponding to a calibration code, as described previously. In some embodiments, the material from second layer 910 may be selected to have a high degree of adhesion to the insulative material 75'. For example, second layer 910 and insulative material 75' may have a higher degree of adhesion than metallic materials used for first layer 900 and insulative material 75'. Therefore, the use of the second layer 910 may further improve adhesion of insulative material 75', thereby preventing abrasion of insulative material 75', and preventing erroneous readings due to loss of insulative material 75'.

Other embodiments of the invention will be apparent to those skilled in the art from consideration of the specification and practice of the invention disclosed herein. It is intended that the specification and examples be considered as exemplary only, with a true scope of the invention being indicated by the following claims.

## Claims

1. A diagnostic test strip (10) including a proximal connecting end (12) and a distal end (14), the test strip (10) comprising:
at least one electrically insulating substrate layer (16);
a plurality of electrical strip contacts (15) disposed on the at least one insulating
substrate layer (16) near the distal end (14) of the test strip (10),
at least one electrode (19) disposed on the at least one insulating substrate layer (16) near the proximal end (12) of the test strip (10);
conductive traces (17) electrically connecting the electrodes (19) to at least some of the electrical strip contacts (15); and
a reagent layer contacting at least a portion of at least one electrode (19),
wherein the electrical strip contacts (15) include a first conductive layer (900) disposed on the substrate (16) and a second conductive layer (910) disposed on top of the first conductive layer (900), wherein the first conductive
layer (900) includes a conductive metallic material **characterised in that** the second conductive
layer (910) includes a carbon-based material.

2. The diagnostic test strip of claim 1, wherein the first conductive layer (900) includes gold.

3. The diagnostic test strip of claim 1, wherein the second conductive layer (910) includes a carbon film, or a silver-carbon film.

4. A method of making a plurality of test strips (10), said method comprising:
selecting at least one electrically insulating substrate material (16);
applying a first conductive layer (900) including a conductive metallic material to at least a portion of the substrate to produce at least one electrical strip contact (15);
applying a second conductive layer (910) including a carbon-based material on top of the first conductive layer (900) ; and
applying an insulating material on top of the second conductive layer (910) in a pattern corresponding to test strip calibration information.

5. The method of claim 4, wherein the second conductive layer (910) includes a carbon film, or a silver-carbon film.

6. The method of claim 4, wherein the second conductive layer (910) is produced using a screen-printing process, a flexographic-printing process, a gravure-printing process, an ink-jet printing process, or a spray-printing process.

7. The diagnostic test strip of claim 1, further comprising:
a semiconductive layer disposed between the first conductive layer and the second conductive layer.

8. The diagnostic test strip of claim 7, wherein the semiconductive layer includes indium-zinc oxide.

## Patentansprüche

1. Diagnostischer Teststreifen (10), aufweisend ein proximales Anschlussende (12) und ein distales Ende (14), wobei der Teststreifen (10) umfasst:
mindestens eine elektrisch isolierende Substratschicht (16);
eine Vielzahl von elektrischen Streifenkontakten (15), die auf der mindestens einen isolierenden Substratschicht (16) in der Nähe des distalen Endes (14) des Teststreifens (10) angeordnet sind,
mindestens eine Elektrode (19), die auf der mindestens einen isolierenden Substratschicht (16) in der Nähe des proximalen Endes (12) des Teststreifens (10) angeordnet ist;
leitende Spuren (17), die die Elektroden (19) an mindestens einige der elektrischen Streifenkontakte (15) elektrisch anschließen; und
eine mit mindestens einem Teil der mindestens einen Elektrode (19) in Kontakt stehende Reagenzschicht, wobei die elektrischen Streifenkontakte (15) eine erste auf dem Substrat (16) angeordnete Leiterschicht (900) und eine zweite auf der ersten Leiterschicht (900) angeordnete Leiterschicht (910) aufweisen, wobei die erste Leiterschicht (900) ein leitfähiges metallisches Material aufweist, **dadurch gekennzeichnet, dass** die zweite Leiterschicht (910) ein auf Kohlenstoff basierendes Material aufweist.

2. Diagnostischer Teststreifen nach Anspruch 1, wobei die erste Leiterschicht (900) Gold aufweist.

3. Diagnostischer Teststreifen nach Anspruch 1, wobei die zweite Leiterschicht (910) eine Kohlenstofffolie oder eine Silber-Kohlenstofffolie aufweist.

4. Verfahren zum Herstellen einer Vielzahl von Teststreifen (10), wobei das Verfahren umfasst:
Auswählen mindestens eines elektrisch isolierenden Substratmaterials (16);
Auftragen einer ersten Leiterschicht (900), die ein leitfähiges metallisches Material aufweist, auf mindestens einen Teil des Substrats, um mindestens einen elektrischen Streifenkontakt (15) herzustellen;
Auftragen einer zweiten Leiterschicht (910), die ein auf Kohlenstoff basierendes Material aufweist, auf die erste Leiterschicht (900); und
Auftragen eines isolierenden Materials auf die zweite Leiterschicht (910) in einem der Kalibrierungsinformation des Teststreifens entsprechenden Muster.

5. Verfahren nach Anspruch 4, wobei die zweite Leiterschicht (910) eine Kohlenstofffolie oder eine Silber-Kohlenstofffolie aufweist.

6. Verfahren nach Anspruch 4, wobei die zweite Leiterschicht (910) unter Verwendung eines Siebdruckverfahrens, eines Flexodruckverfahrens, eines Tiefdruckverfahrens, eines Tintenstrahldruckverfahrens oder eines Spritzdruckverfahrens hergestellt wird.

7. Diagnostischer Teststreifen nach Anspruch 1, weiterhin umfassend:
eine Halbleiterschicht, die zwischen der ersten Leiterschicht und der zweiten Leiterschicht angeordnet ist.

8. Diagnostischer Teststreifen nach Anspruch 7, wobei die Halbleiterschicht Indiumzinkoxid aufweist.

## Revendications

1. Bandelette de test de diagnostic (10) comprenant une extrémité proximale de connexion (12) et une extrémité distale (14), la bandelette de test (10) comprenant :
au moins une couche de substrat électriquement isolant (16) ;
une pluralité de contacts électriques de bandelette (15) disposés sur l'au moins une couche de substrat isolant (16) à proximité de l'extrémité distale (14) de la bandelette de test (10),
au moins une électrode (19) disposée sur l'au moins une couche de substrat isolant (16) à proximité de l'extrémité proximale (12) de la bandelette de test (10) ;
des traces conductrices (17) connectant électriquement les électrodes (19) à au moins certains des contacts électriques de bandelette (15) ; et
une couche de réactif en contact avec au moins une partie de l'au moins une électrode (19),
dans laquelle les contacts électriques de bandelette (15) comprennent une première couche conductrice (900) disposée sur le substrat (16) et une seconde couche conductrice (910) disposée au-dessus de la première couche conductrice (900), dans laquelle la première couche conductrice (900) comprend un matériau métallique conducteur **caractérisée en ce que** la seconde couche conductrice (910) comprend un matériau à base de carbone.

2. Bandelette de test de diagnostic selon la revendication 1, dans laquelle la première couche conductrice (900) comprend de l'or.

3. Bandelette de test de diagnostic selon la revendication 1, dans laquelle la seconde couche conductrice (910) comprend un film de carbone, ou un film d'argent-carbone.

4. Procédé de préparation d'une pluralité de bandelettes de test (10), ledit procédé comprenant :
la sélection d'au moins un matériau de substrat électriquement isolant (16) ;
l'application d'une première couche conductrice (900) comprenant un matériau métallique conducteur sur au moins une partie du substrat afin de produire au moins un contact électrique de bandelette (15) ;
l'application d'une seconde couche conductrice (910) comprenant un matériau à base de carbone au-dessus de la première couche conductrice (900) ; et
l'application d'un matériau isolant au-dessus de la seconde couche conductrice (910) selon un motif correspondant à des informations d'étalonnage de bandelette de test.

5. Procédé selon la revendication 4, dans lequel la seconde couche conductrice (910) comprend un film de carbone, ou un film d'argent-carbone.

6. Procédé selon la revendication 4, dans lequel la seconde couche conductrice (910) est produite en utilisant un processus de sérigraphie, un processus d'impression flexographique, un processus d'héliogravure, un processus d'impression par jet d'encre, ou un processus d'impression par pulvérisation.

7. Bandelette de test de diagnostic selon la revendication 1, comprenant en outre :
une couche semi-conductrice disposée entre la première couche conductrice et la seconde couche conductrice.

8. Bandelette de test de diagnostic selon la revendication 7, dans laquelle la couche semi-conductrice comprend un oxyde d'indium et de zinc.
